# NEW EUROPEAN PATENT SPECIFICATION

(11) **EP 0 717 041 B2**
(45) Date of publication and mention of the opposition decision: **15.08.2007**
(45) Mention of the grant of the patent: 07.08.2002
(21) Application number: 95119595.7
(22) Date of filing: 12.12.1995
(51) Int. Cl.: C07D 305/14

(54) **Crystalline paclitaxel hydrate**
Kristallines Paclitaxel-Hydrat
Hydrate de paclitaxel cristallin

(30) Priority: 13.12.1994 US 355125
(43) Date of publication of application: 19.06.1996
(73) Proprietor: Bristol-Myers Squibb Company, Princeton, NJ 08543-4000 (US)
(72) Inventor: Perrone, Robert K., Liverpool, NY (US); Stenberg, Scott R., Baldwinsville, NY 13027 (US); Kaplan, Murray A., Syracuse, NY (US); Saab, Akram, Beirut (LB); Agharkar, Shreeram, Fayetteville, NY (US)
(74) Representative: Kinzebach, Werner

(56) References cited:
- EP-A- 0 617 018
- WO-A-92/07842
- US-A- 4 814 470
- US-A- 4 960 790
- CHEMICAL ABSTRACTS, vol. 113, no. 75, 1990 Columbus, Ohio, US; abstract no. 142641k, GUERITTE-VOEGELEIN,F ET AL. 'STRUCTURE OF A SYNTHETIC TAXOL PRECURSOR.' page 723; & ACTA CRYSTALLOGR. SECT. C:CRYST. STRUCT. COMMUN., vol. 46, no. 5, 1990 ENGL, pages 781-784,
- CHEMICAL ABSTRACTS, vol. 123, no. 1, 1995 Columbus, Ohio, US; abstract no. 132019f, MASTROPAOLO,D. ET AL. 'CRYSTAL AND MOLECULAR STRUCTURE OF PACLITAXEL(TAXOL).' page 29; & PROC. NATL. ACAD. SCI., vol. 92, no. 15, 1995 U.S.A., pages 6920-6924,
- American Journal of Hospital Pharmacists; 1991, pages 1520-1524
- National Cancer Institute; 1983, Clinical brochure NSC 125973
- Journal of Organic Chemistry - 1981, pages 1469-1474
- National Cancer Institute;1991 (revised), Clinical brochure NSC 125973
- Journal of the American Chemical Society; 1971, pages 2325-2327
- Life Magazine; 1992, pages 71-76
- E-mail from Professor Elsohly; 2002
- Affidavit and C.V. by and for Dr. McChesney

## Description

The present invention concerns a new pseudopolymorph of paclitaxel, a pharmaceutical composition thereof, and its use as an antitumor agent. Another aspect relates to preparation of this pseudopolymorph.

Taxol® (paclitaxel) is a natural product extracted from the bark of Pacific yew trees, Taxus brevifolia. It has been shown to have excellent antitumor activity in in vivo animal models, and recent studies have elucidated its unique mode of action, which involves abnormal polymerization of tubulin and disruption of mitosis. It has been recently approved for the treatment of ovarian cancer; and studies involving breast, colon, and lung cancers have shown promising results. The results of paclitaxel clinical studies are reviewed by Rowinsky and Donehower in "The Clinical Pharmacology and Use of Antimicrotubule Agents in Cancer Chemotherapeutics" Pharmac. Ther., 52:35-84, 1991; and by K.C. Nicolaou et al. in "Chemistry and Biology of Paclitaxel" Angew. Chem., Int. Ed. Engl., 33: 15-44, 1994, and also in the references cited therein.

During our work directed at paclitaxel development, we discovered that paclitaxel can exist in at least two crystalline forms under ambient conditions. One form (coded Form A) is an anhydrous form and, to our knowledge, it is the only form reported in the literature to date. Form A is substantially anhydrous at 33% RH and 75% RH at room temperature. Another form (coded Form B) is a hydrated form heretofore unknown, and recently discovered serendipitously by our group. Form B contains about 7.3% water at 33% RH and 8.5% water at 75% RH at room temperature pointing to being a tetrahydrate. Upon drying under vaccum at 25-50°C, the water content of Form B drops to about 3.5 to 4.0 % pointing to being a dihydrate. Thus, the present invention concerns a new pseudopolymorph (Form B) of paclitaxel containing about four moles of water. The invention further provides a pharmaceutical compositions of paclitaxel Form B, and its use as antitumor agent.

The present invention concerns a new pseudopolymorph of paclitaxel (Form B), a pharmaceutical composition thereof, and its use as an antitumor agent. Another aspect relates to preparation of this pseudopolymorph.
- Figure 1.: XRD powder pattern of paclitaxel Form A.
- Figure 2.: DSC curve of paclitaxel Form A.
- Figure 3.: Mid-IR spectrum of paclitaxel Form A.
- Figure 4.: Expansion of the high frequency region of the IR spectrum of paclitaxel Form A prepared by the mineral oil mull technique.
- Figure 5.: Mid-IR spectrum of paclitaxel Form A prepared by: (a) KBr pellet (b) mineral oil mull.
- Figure 6.: XRD powder pattern of paclitaxel Form B.
- Figure 7.: DSC curve of paclitaxel Form B.
- Figure 8.: TG curve of paclitaxel Form B.
- Figure 9.: DSC curve of paclitaxel Form B, heated at 3°C/minute.
- Figure 10.: Mid-IR spectrum of paclitaxel Form B prepared by the mineral oil mull technique.
- Figure 11.: Expansion of the high frequency region of the IR spectrum of paclitaxel Form B prepared by the mineral oil mull technique.
- Figure 12.: Mid-IR spectra of paclitaxel Form B prepared by (a) KBR pellet (b) mineral oil mull.

Highly water insoluble crystalline paclitaxel (Form A) exists primarily as an anhydrate (sand-like crystals). It is substantially non-hygroscopic under normal laboratory relative-humidities (RH approximately 50-60%; 20-30°C). However, when contacted with moisture at very high relative humidities (90-100% RH) or as in aqueous suspensions, dispersions or emulsions, the anhydrate (Form A) can (will) convert (as a function of time, temperature, agitation, etc.) to a thermodynamically more stable, pseudopolymorphic hydrated form containing about 2-4 moles of water. These hydrated crystals are of a fine, hair-like morphology and even less water soluble than the highly insoluble parent anhydrate counterpart, Form A.

Form B can be readily prepared by dissolving Form A anhydrate in solvent as acetone, ethanol, acetonitrile, N,N-dimethylformamide, etc., and crystallization by addition to water. The water content of the Form B, after vacuum drying at 25°C, indicates a very strong probability for a dihydrate. There is evidence that a laboratory air-dried sample of the hydrate (RH=50%, T=25°C) appeared to be a tetrahydrate. However, vacuum drying at 25°C readily removes the 2-moles of labile water, yielding a stable dihydrate.

The importance (chemical, physical, biological and especially patentability) of the hydrated paclitaxel Form B rests primarily (but not exclusively) in aqueous contact as suspensions, emulsions, etc. Here thermodynamically less stable anhydrate Form A can (will) convert to the Form B hydrate with changes in physical-chemical properties. Thus with dosage form work requiring aqueous contact with paclitaxel (emulsions, suspensions, dispersion, etc.), it is required that Form B be used. This eliminates any untoward Form A conversion to Form B. Thus, in at least water contacted dosage forms (and in many instances, dry fills) hydrated paclitaxel forms could be of much more importance than the thermodynamically less stable anhydrate (Form A) counterpart. Inevitably, the water contacted anhydrate (Form A) will convert, all or in part to a hydrated form. Thus a preformed hydrate is the paclitaxel form of choice at least in aqueous environments. Also, as a powder (bulk or in capsule or tablet, etc. formulations), paclitaxel anhydrate (Form A) could convert untowardly (partially or completely) to a hydrated form as a function of the moisture present.

### Characterization

X-ray powder diffraction (XRD), differential scanning calorimetry (DSC), thermogravimetry (TG), and infrared (IR) spectroscopy were used to further characterize the two forms.

### X-Ray Powder Diffraction

X-ray powder diffraction patterns were collected on a Rigaku microdiffractometer, model PSPC-MPD. The powders were packed in 0.5 mm glass capillary tubes and were analyzed according to a standard method in the art. The x-ray generator (Philips model XRG 31000) was operated at 45 kV and 40 mA, using the copper Kα line as the radiation source. Each sample was rotated along the chi axis and data was collected between 0 and 150 deg 2-theta A collection time of 3000 sec was used.

### DSC/TG

Measurements of differential scanning calorimetry and thermogravimetry were obtained on a DuPont model 2100 thermal analysis system. Approximately 3 mg samples were accurately weighed into a DSC pan. The pans were hermetically sealed and a pinhole was punched into the pan lid. The use of the pinhole allows for pressure release, but still assures that the thermal reactions proceed under controlled conditions. The samples were cooled to approximately 10°C and then heated at a rate of 10°C/min, up to a final temperature of 300°C. The DSC data were obtained following a standard method in the art. For TG determinations, approximately 10 mg of sample were placed on the pan and inserted into the TG furnace. The samples were heated at a rate of 10°C/min, up to a final temperature of 200°C. The TG data were obtained using a standard method in the art.

### Moisture Uptake Rate

The room temperature (RT) moisture uptake/loss rates were determined at 33%, 75%, and 100% relative humidity (RH) (in steps) on a Cahn Digital Recording Balance fitted with a system to maintain and monitor relative humidity. The system consists of a glass tube enclosing the sample, a saturation chamber for salt solutions, a peristaltic pump to recirculate the saturated air, and an in-line chilled mirror dew point-%RH meter. The system is equilibrated to the desired %RH and sample is added through the access port. Samples were run until the uptake rate leveled, the salt reservoir was changed and the experiment was resumed at the new relative humidity. This process was repeated as necessary for the absorption and desorption.

### Hot Stage Microscopy

A Mettler model FP-80 controller with heating stage was used on a Wilde-Lietz polarizing light microscope. A few particles were placed on a glass slide and observed at 250X while heating at 10°C per minute.

### IR Spectroscopy

Their IR spectra of paclitaxel were obtained by either the KBr pellet and/or mineral oil mull preparation techniques.

### Paclitaxel Form A

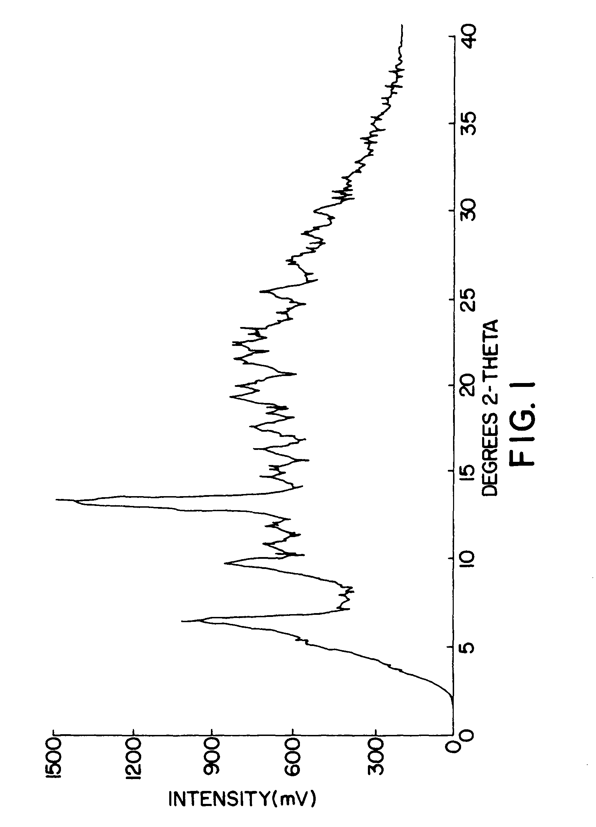

The XRD, DSC, and IR curves of paclitaxel Form A are shown in Figures 1-5, respectively.

The x-ray powder diffraction pattern for Form A (Figure 1) show a crystalline structure with major peaks at approximately 6.4, 9.8, and 13.2 deg 2-theta. The amorphous background observed in the plot is due to the glass capillary tube holding the powder.

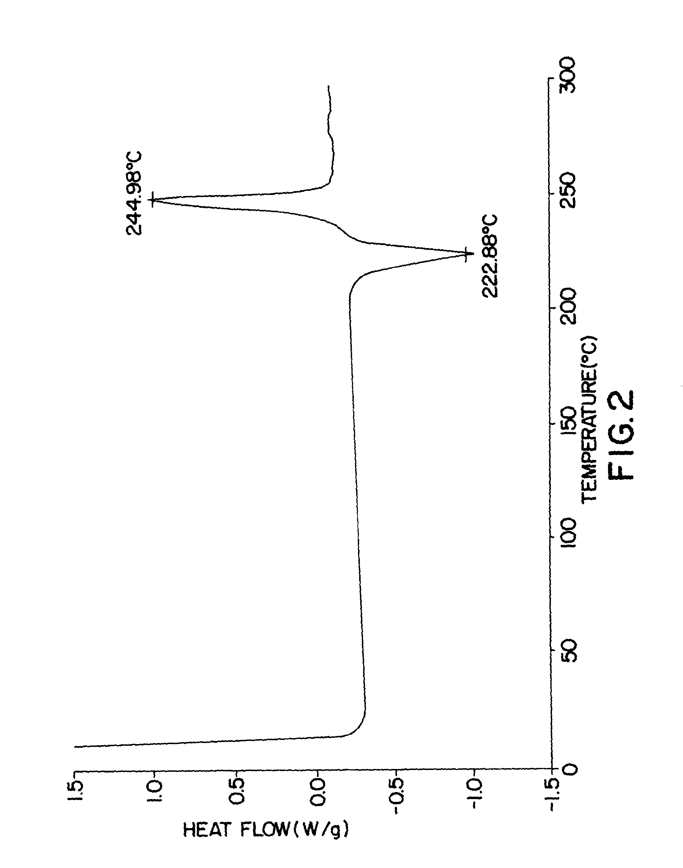

The DSC curve of Form A (Figure 2) exhibits a melting endotherm at approximately 223°C, with subsequent decomposition at approximately 245°C. The integrated melting endotherm has a heat of fusion (ΔH_{f}) of approximately 41 J/g. This behavior was confirmed with hot stage microscopy where the melting point was observed at 214°C.

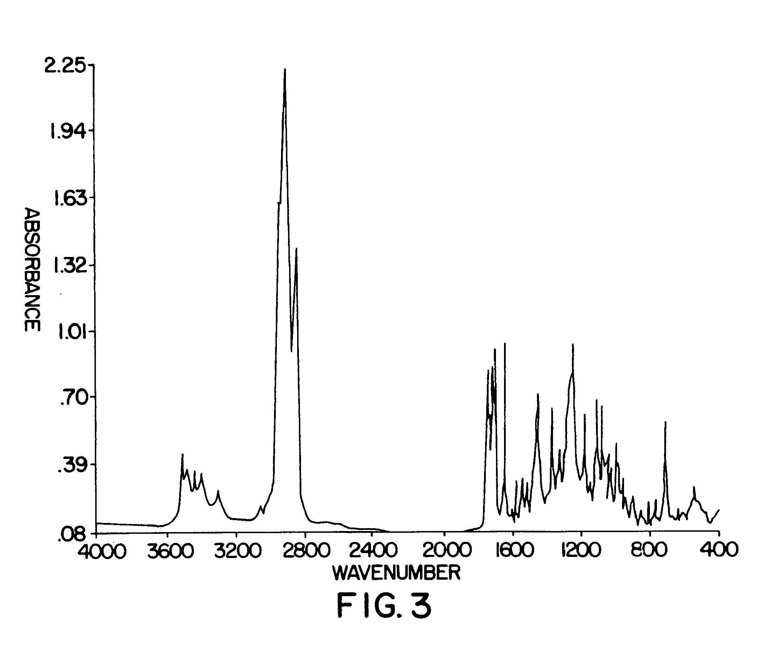

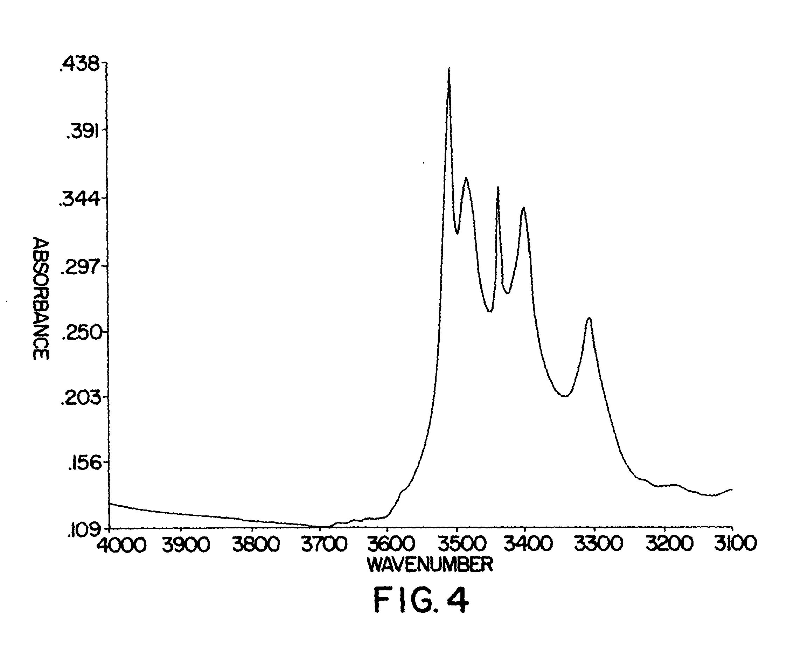

The mid-IR spectrum of Form A is shown in Figure 3. This spectrum was obtained by the mineral oil mull technique so that the crystal form would not be destroyed during sample preparation. In the high frequency region (Figure 4), a series of sharp absorption bands are noted at 3513, 3486, 3441, 3402 and 3309 cm⁻¹. A small absorption band is also noted at 3576 cm^{-1.} The band centered at 3309 cm⁻¹ is assigned as the N-H stretch, whereas the multiple bands from 3520 to 3350 cm⁻¹ are assigned to the various O-H moieties.

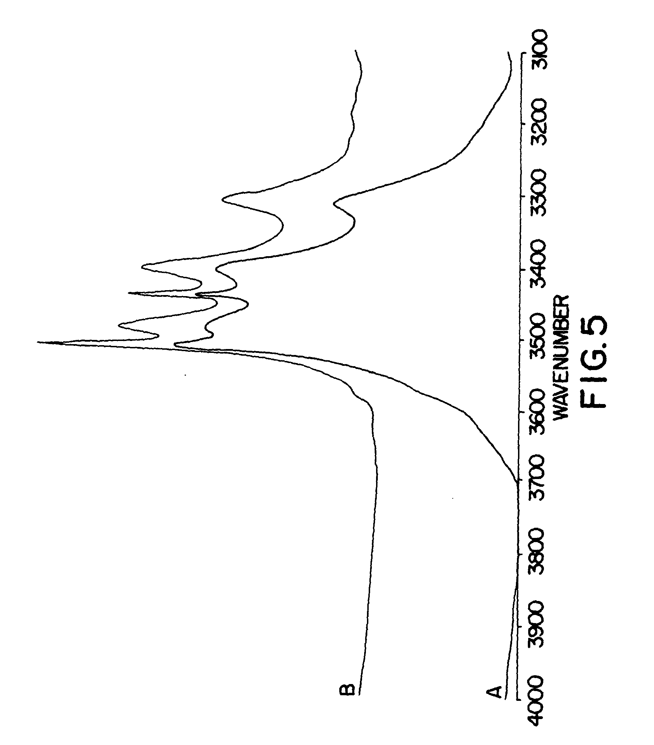

The IR spectrum of the A form obtained by the KBr pellet technique and the mineral oil mull technique is shown in Figure 5. Comparison of the spectra obtained by the two techniques indicates that no differences in the absorption frequencies are noted between the two spectra. This seems to indicate that the high pressure required to prepare the KBr pellet does not destroy the Form A character.

Paclitaxel form A is crystalline and anhydrous based on the above data, and it picks up less that 1% moisture between 6 and 70% relative humidity but does sorb moisture at 100%RH.

### Paclitaxel Form B

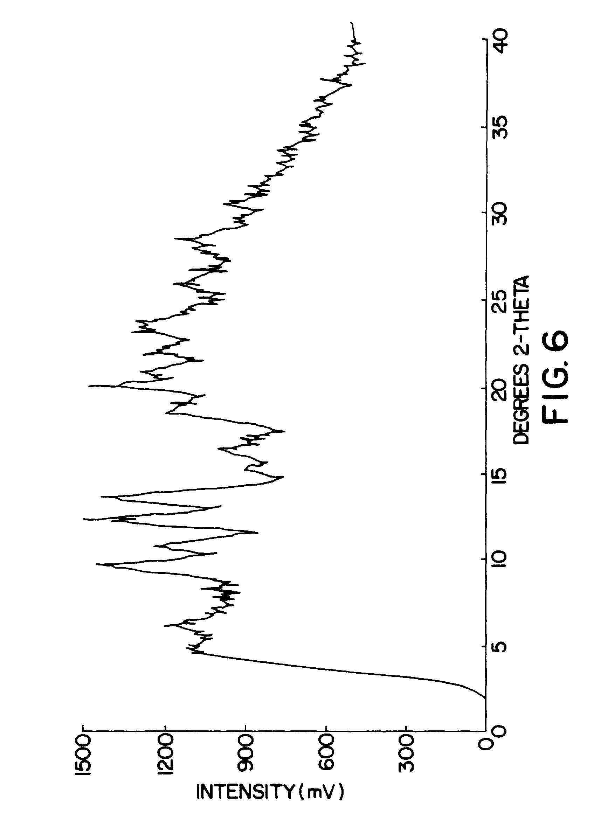

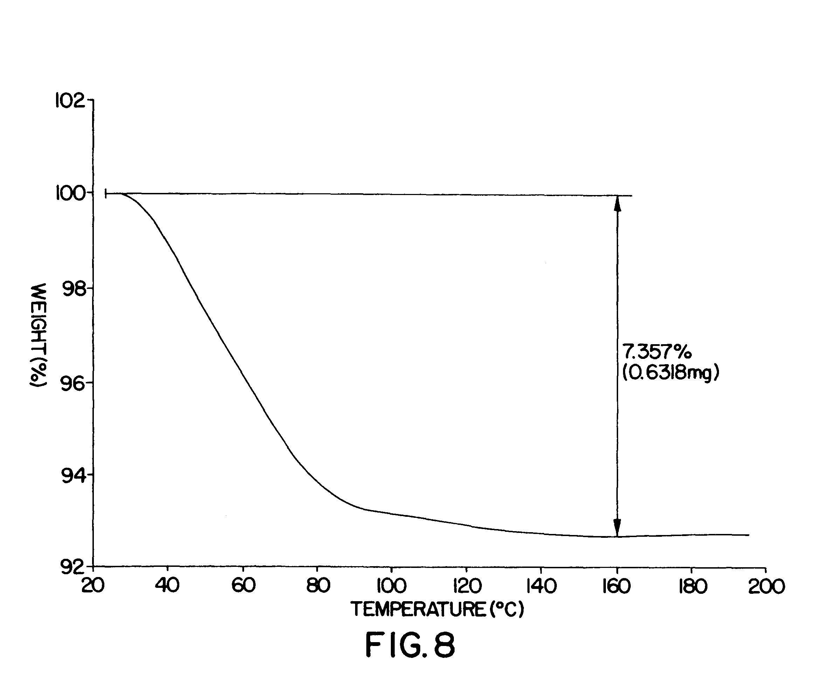

The XRD, DSC, TGA, and IR curves of the form B of paclitaxel are shown in Figures 6-12. One lot exhibited a moisture content of 7.36% (theoretical water content of tetrahydrate: 7.7%), as measured by TG.

The x-ray powder diffraction pattern for paclitaxel Form B (Figure 6) shows a different pattern than observed for Form A. Several reflections present in Form A are absent in Form B (12, 13.2, 17.7, 25.8 deg 2-theta), and several new reflections occur in Form B (12.6, 13.8, 20.5).

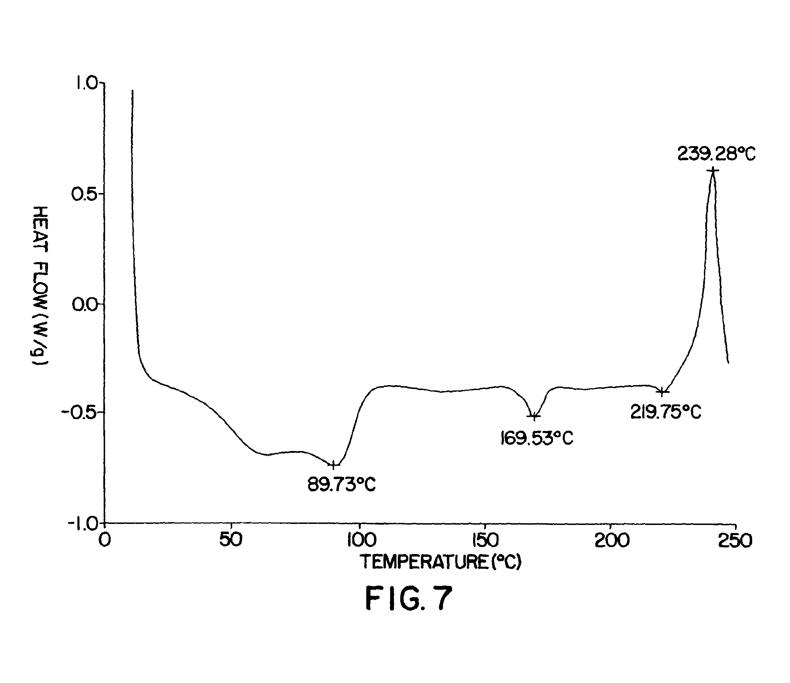

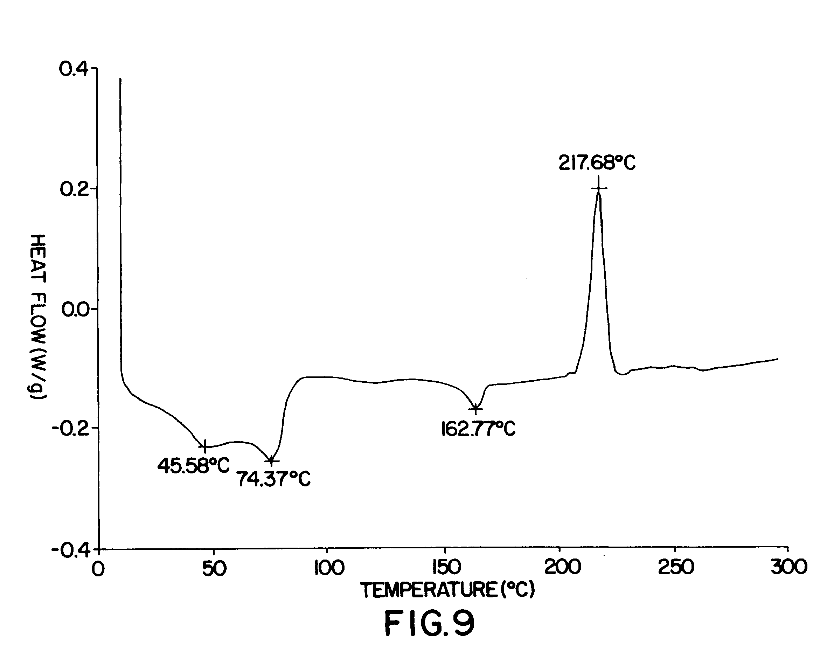

The DSC curve for Form B (Figure 7) exhibits several characteristic events. Beginning immediately after heating, a broad endotherm peaking at approximately 60°C is observed. A second partially resolved endotherm peaks at approximately 90°C. Both of these correspond to the approximately 7% weight loss (theoretical water content of tetrahydrate: 7.7%) observed in the TG curve. The next event is an endotherm at approximately 170°C which is unaccompanied by weight loss and identified as a melt by hot stage microscopy. This event has a ΔH_{f} of approximately 7 J/g (slightly dependent on heating rate) which is approximately six times less than that associated with Form A. The extent to which the crystallinity is retained following dehydration is not known. Figure 9 shows the DSC curve of the same material run at 3°C per minute to show the heating rate dependence of the thermal events.

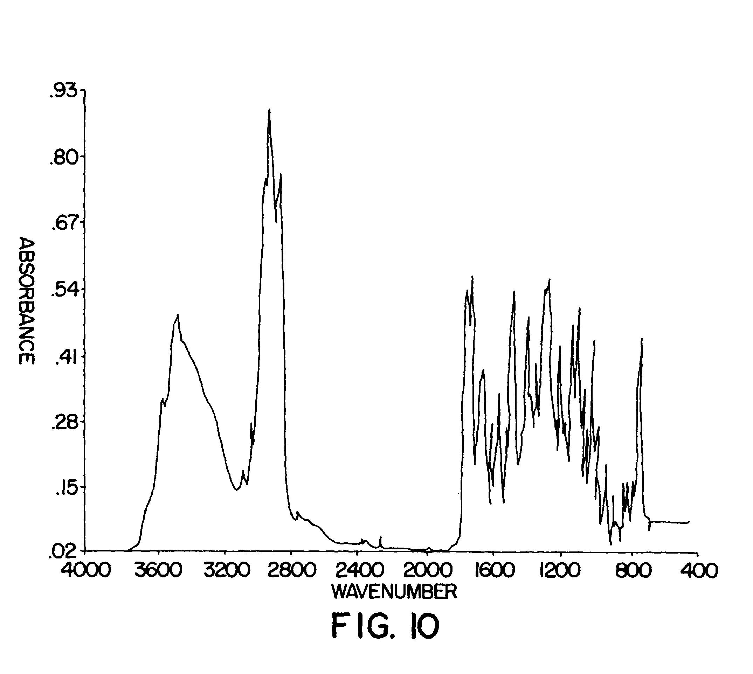

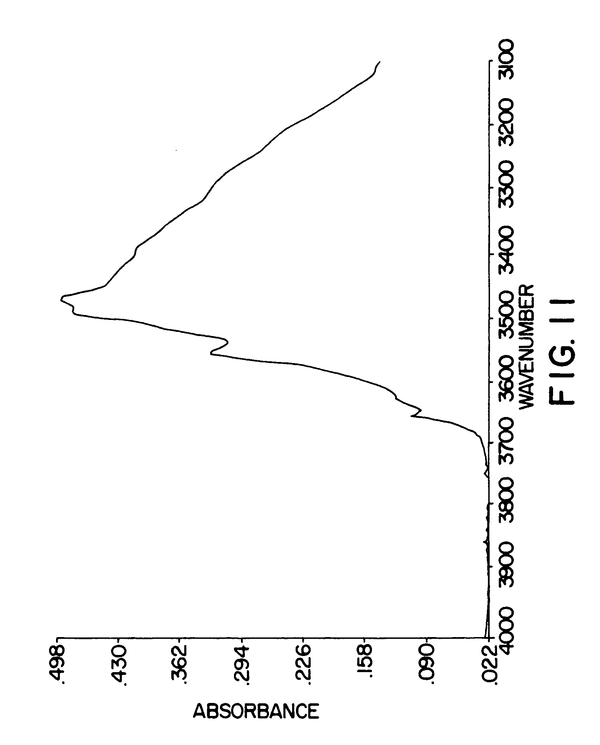

The IR spectrum of paclitaxel Form B is shown in Figure 10. Again, the mineral oil mull technique was used for sample preparation. Upon expansion of the high frequency region of the IR spectrum (Figure 11), differences are noted in the absorption pattern of the B form. A broad underlying absorption band is seen indicating the presence of adventitious water. More importantly, two high frequency absorption bands are detected at 3553 and 3655 cm⁻¹. Due to the sharp, narrow bandwidth and high frequency of these two absorption bands, they are assigned as crystalline water O-H stretching frequencies. Due to the unique frequency of these two absorption bands, they can be used as diagnostic peaks for the presence of the B form of paclitaxel.

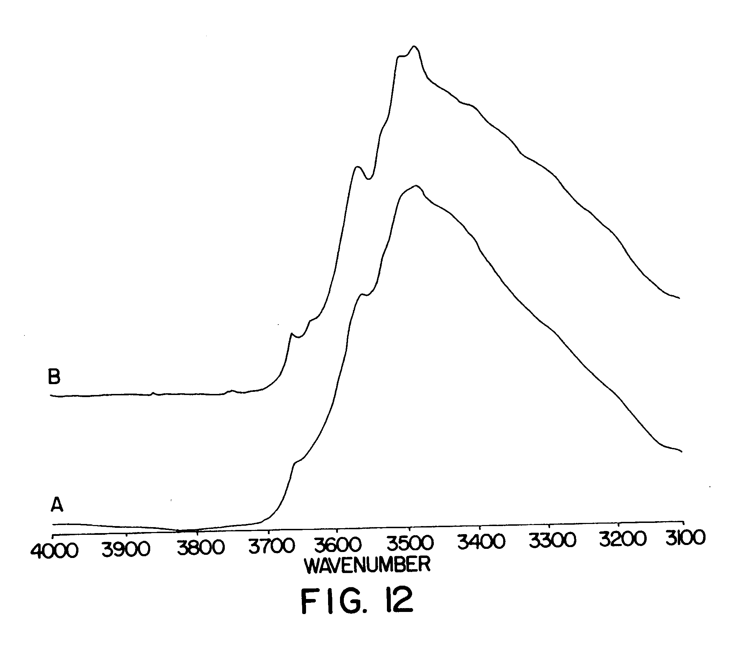

In Figure 12, the IR spectra of the B form prepared by the two sampling techniques are overlaid. Again, no differences are noted in the absorption frequencies between the two spectra indicating that the high pressure used to prepare the KBr pellet does not destroy the B form.

These data indicate that paclitaxel Form B is a crystalline hydrate (pseudopolymorph) that dehydrates over a temperature range of 90°C to 130°C below the apparent melting point of approximately 170°C.

Crystalline paclitaxel has been reported in many places. EP 0 617 018 A1 describes the preparation of paclitaxel. The products obtained in examples 6, 7 and 8 are said to contain 1.0, 1.3 and 2.55 moles of water, respectively. Journal of Natural Products, Vol. 47, No. 1,1984, p 136; J. Org. Chem., Vol 46, No. 7, 1981, p. 1473; PCT publication WO 92/07842, p 19, published May 14, 1992; and J. Am. Chem. Soc., 1971, 93, p. 2325 report crystalline paclitaxel having melting points in the range of 205-208, 198-203, and 212-214, and 213-216°C, respectively, all pointing to Form A.

In summary, paclitaxel exists in at least two stable crystalline forms under ambient conditions. Form A is a crystalline anhydrate (melting point = 214°C by hot stage microscopy, ΔH_{f} ≈ 41 J/g) with a characteristic powder XRD pattern and IR spectrum. Form B is a crystalline hydrate (dehydration from ambient to approximately 100°C followed by an apparent melting point ≈ 168°C, ΔH_{f} ≈ 7 J/g) with a characteristically different powder XRD and IR spectrum from Form A.

The present invention also provides a process for producing paclitaxel Form B which comprises adding an organic solution of Form A into water and crystallizing Form B, or contacting Form A with water.

### SPECIFIC EMBODIMENTS

The following procedures are two representative preparations (but not limiting) for crystallin solids having a water content close to dihydrate.

### Example 1.

Transfer paclitaxel solids (Form A, irregularly shaped crystals) to a Type I glass vial and add appropriate volume of Milli-Q water to give an ∼ 20 mg/mL suspension (e.g. lg/50 mL). Briefly sonicate to disperse solids, then vigorously stir the suspension at room temperature (∼ 23 °C) for ∼ 72 hours. Collect the very fine hair/needle-like crystals by vacuum (house) filtration on a medium (10-20 micron) sintered glass filter and wash several times with water. The crystals are then dried at ∼ 25 °C using high vacuum for ∼ 24 hours. Yield is substantially quantitative.

### Example 2.

Transfer paclitaxel solids (Form A, irregularly shaped crystals) to a Type I glass vial and add appropriate volume of ethyl alcohol (100%) to give an ∼ 40 mg/mL solution (e.g. lg/25 mL). Sonicate and stir to obtain pale straw yellow-colored solution. Slowly add the paclitaxel solution dropwise to a 10-fold volume of vigorously stirring Milli-Q water (e.g. 250 mL) at room temperature (∼ 23 °C), yielding a white suspension of very fine hair/needle-like crystals. Continue stirring the suspension of crystals in a sealed glass container ∼ 24 hours at ∼ 23 °C. Filter the suspension using a medium (10-20 micron) sintered glass filter with house vacuum, and wash the crystalline solid cake several times with water. The crystals are then dried using high vacuum at ∼ 23 °C for 24 hours. Yield is >90%.

Once crystals are obtained, it is preferable to add them as seed crystals to enhance subsequent onset and rate of conversion from Form A to Form B, since in some instances conversion Form A to Form B may not be affected in the absence of seed crystals.

Like paclitaxel Form A, Form B displays a significant inhibitory effect with regard to abnormal cell proliferation, and has therapeutic properties that make it possible to treat patients who have pathological conditions associated with an abnormal cell proliferation, thus is useful in human and/or veterinary medicine. The pathological conditions include the abnormal cellular proliferation of malignant or non-malignant cells in various tissues and/or organs, including, non-limitatively, muscle, bone, and/or conjunctive tissues; the skin, brain, lungs, and sexual organs; the lymphatic and/or renal system; mammary cells and/or blood cells; the liver, digestive system, and pancreas; and the thyroid and/or adrenal glands. These pathological conditions can also include psoriasis; solid tumors; ovarian, breast, brain, prostate, colon, stomach, kidney, and/or testicular cancer; Kaposi's sarcoma; cholangiocarcinoma; choriocarcinoma; neuroblastoma; Wilm's tumor; Hodgkin's disease; melanomas; multiple myelomas; chronic lymphocytic leukemias; and acute or chronic granulocytic lymphomas. Form B in accordance with the invention is particularly useful in the treatment of ovarian cancer. Thus, another aspect of the instant invention concerns a method for inhibiting human and/or other mammalian tumors which comprises administering to a tumor bearing host an antitumor effective amount of paclitaxel Form B.

Paclitaxel Form B of the present invention may be used in a manner similar to that of paclitaxel Form A; therefore, an oncologist skilled in the art of cancer treatment will be able to ascertain, without undue experimentation, an appropriate treatment protocol for administering a compound of the present invention. The dosage, mode and schedule of administration for compounds of this invention are not particularly restricted, and will vary with the particular compound employed. Thus Form B of the present invention may be administered via any suitable route of administration, preferably parenterally as a suspension or in solution; the dosage may be, for example, in the range of about 1 to about 100 mg/kg of body weight, or about 20 to about 500 mg/m². The actual dose used will vary according to the particular composition formulated, the route of administration, and the particular site, host and type of tumor being treated. Many factors that modify the action of the drug will be taken into account in determining the dosage including age, weight, sex, diet and the physical condition of the patient.

The present invention also provides pharmaceutical compositions (formulations) containing an antitumor effective amount of Form B in combination with one or more pharmaceutically acceptable carriers, excipients, diluents or adjuvants. Examples of formulating Form A may be found in, for example, United States Patents Nos. 4,960,790 and 4,814,470, and such examples may be followed to formulate Form B of this invention. For example, Form B invention may be formulated in the form of tablets, pills, powder mixtures, capsules, injectables, solutions, suspensions, suppositories, emulsions, dispersions, food premix, and in other suitable forms. It may also be manufactured in the form of sterile solid compositions, for example, freeze dried and, if desired, combined with other pharmaceutically acceptable excipients. Such solid compositions can be reconstituted with sterile water, physiological saline, or a mixture of water and an organic solvent, such as propylene glycol, ethanol, and the like, or some other sterile injectable medium immediately before use for parenteral administration as a suspension (microdispersion) or in solution.

Typical of pharmaceutically acceptable carriers are, for example, manitol, urea, dextrans, lactose, potato and maize starches, magnesium stearate, talc, vegetable oils, polyalkylene glycols, ethyl cellulose, poly(vinylpyrrolidone), calcium carbonate, ethyl oleate, isopropyl myristate, benzyl benzoate, sodium carbonate, gelatin, potassium carbonate, silicic acid. The pharmaceutical preparation may also contain nontoxic auxiliary substances such as emulsifying, preserving, wetting agents, and the like as for example, sorbitan monolaurate, triethanolamine oleate, polyoxyethylene monostearate, glyceryl tripalmitate, dioctyl sodium sulfosuccinate, and the like.

## Claims

1. Paclitaxel hydrate containing about four moles of water.

2. Paclitaxel hydrate having water content of about 7.3-8.5 %.

3. Paclitaxel hydrate having substantially the same DSC curve as set out in Fig. 7.

4. Paclitaxel hydrate having substantially the same X-ray diffractogram as set out in Fig. 6 and substantially the same IR spectrum, in oil mull, as set out in Fig. 10.

5. Paclitaxel hydrate having substantially the same X-ray diffractogram as set out in Fig. 6, and substantially the same IR spectrum, in oil mull, as set out in Fig. 10, and substantially the same DSC curve as set out in Fig. 7.

6. A pharmaceutical composition comprising an effective antitumor amount of paclitaxel hydrate having substantially the same X-ray diffractogram as set out in Fig. 6, and substantially the same IR spectrum, in oil mull, as set out in Fig. 10, together with pharmaceutically acceptable carrier or diluent.

7. A pharmaceutical composition comprising an effective antitumor amount of paclitaxel hydrate having substantially the same X-ray diffractogram as set out in Fig. 6, and substantially the same IR spectrum, in oil mull, as set out in Fig. 10, and substantially the same DSC curve as set out in Fig. 7, together with pharmaceutically acceptable carrier or diluent.

8. The use of a paclitaxel hydrate as defined in any one of claims 1 to 5 for preparing a pharmaceutical composition for treating tumors.

## Patentansprüche

1. Paclitaxel-Hydrat, enthaltend etwa vier Mol Wasser.

2. Paclitaxel-Hydrat mit einem Wassergehalt von etwa 7,3 bis 8,5%.

3. Paclitaxel-Hydrat mit im Wesentlichen der in Figur 7 dargestellten DSC-Kurve.

4. Paclitaxel-Hydrat mit im Wesentlichen dem in Figur 6 dargestellten Röntgendiffraktogramm und im Wesentlichen dem in Figur 10 dargestellten IR-Spektrum in öliger Aufschlämmung.

5. Paclitaxel-Hydrat mit im Wesentlichen dem in Figur 6 dargestellten Röntgendiffraktogramm, im Wesentlichen dem in Figur 10 dargestellten IR-Spektrum in öliger Aufschlämmung und im Wesentlichen der in Figur 7 dargestellten DSC-Kurve.

6. Pharmazeutische Zusammensetzung mit einem Zusatz einer antitumoral wirksamen Menge von Paclitaxel-Hydrat mit im Wesentlichen dem in Figur 6 dargestellten Röntgendiffraktogramm und im Wesentlichen dem in Figur 10 dargestellten IR-Spektrum in öliger Aufschlämmung, zusammen mit einem pharmazeutisch akzeptablen Träger oder Verdünnungsmittel.

7. Pharmazeutische Zusammensetzung mit einem Zusatz einer antitumoral wirksamen Menge von Paclitaxel-Hydrat mit im Wesentlichen dem in Figur 6 dargestellten Röntgendiffraktogramm, im Wesentlichen dem in Figur 10 dargestellten IR-Spektrum in öliger Aufschlämmung und im Wesentlichen der in Figur 7 dargestellten DSC-Kurve, zusammen mit einem pharmazeutisch akzeptablen Träger oder Verdünnungsmittel.

8. Verwendung eines Paclitaxel-Hydrats gemäß Definition in einem der Ansprüche 1 bis 5 zur Herstellung einer pharmazeutischen Zusammensetzung zur Behandlung von Tumoren.

## Revendications

1. Hydrate de paclitaxel contenant environ quatre moles d'eau.

2. Hydrate de paclitaxel ayant une teneur en eau d'environ 7,3 - 8,5%.

3. Hydrate de paclitaxel ayant substantiellement la même courbe DSC qu'indiqué dans la figure 7.

4. Hydrate de paclitaxel ayant substantiellement le même diffractogramme de rayons X qu'indiqué dans la figure 6 et substantiellement le même spectre IR, en poussière d'huile, que celui indiqué dans la figure 10.

5. Hydrate de paclitaxel ayant substantiellement le même diffractogramme de rayons X qu'indiqué dans la figure 6 et substantiellement le même spectre IR, en poussière d'huile, comme indiqué dans la figure 10, et substantiellement la même courbe DSC que celle indiquée dans la figure 7.

6. Composition pharmaceutique ayant une quantité effective d'anti-tumeur ajoutée d'hydrate de paclitaxel ayant substantiellement le même diffractogramme de rayons X que dans la figure 6 et substantiellement le même spectre IR, en poussière d'huile, que celui indiqué dans la figure 10 ainsi qu'un support ou diluant pharmaceutiquement acceptable.

7. Composition pharmaceutique ayant une quantité effective d'anti-tumeur ajoutée d'hydrate de paclitaxel ayant substantiellement le même diffractogramme de rayons X que celui indiqué dans la figure 6 et substantiellement le même spectre IR, en poussière d'huile, que celui indiqué dans la figure 10 et substantiellement la même courbe DSC que dans la figure 7, ainsi qu'un support ou diluant pharmaceutiquement acceptable.

8. Utilisation d'hydrate de paclitaxel comme défini dans l'une quelconque des revendications 1 à 5 pour préparer une composition pharmaceutique pour traiter les tumeurs.
